(19) Europäisches Patentamt
European Patent Office
Office européen des brevets

(11) Veröffentlichungsnummer: **0 191 345**
**A2**

(12) # EUROPÄISCHE PATENTANMELDUNG

(21) Anmeldenummer: **86101030.4**

(22) Anmeldetag: **25.01.86**

(51) Int. Cl.⁴: **C 07 D 498/14,** C 07 D 265/32, C 07 D 265/36, C 07 D 413/04 // (C07D498/14, 319:00, 265:00, 265:00)

(30) Priorität: **09.02.85 DE 3504480**

(43) Veröffentlichungstag der Anmeldung: **20.08.86 Patentblatt 86/34**

(84) Benannte Vertragsstaaten: **CH DE FR GB LI**

(71) Anmelder: **BAYER AG, Konzernverwaltung RP Patentabteilung, D-5090 Leverkusen 1 Bayerwerk (DE)**

(72) Erfinder: **Haas, Peter, Dr., Zwengenberger Strasse 43, D-5657 Haan 1 (DE)**

(54) **Neue Cycloacetale, Verfahren zu ihrer Herstellung und ihre Verwendung zur Herstellung Azalactonen.**

(57) Neue polycyclische Acetale der Formel

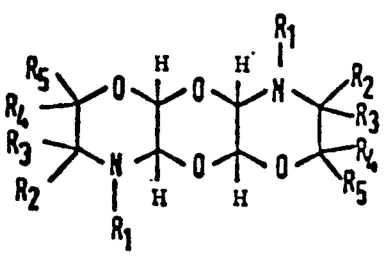

in der
$R_1$ für einen gegebenenfalls substituierten Alkyl-, Cycloalkyl-, Aralkyl-, Aryl- oder Hetaryl-Rest steht und
$R_2$, $R_3$, $R_4$ und $R_5$ unabhängig voneinander Wasserstoff, einen gegebenenfalls substituierten Alkyl-, Cycloalkyl-, Aralkyl- oder Aryl-Rest bedeuten, oder $R_3$ und $R_4$ zusammen einen gegebenenfalls substituierten $C_2$-$C_6$-Alkylen-Rest bilden oder $R_2$, $R_3$, $R_4$ und $R_5$ zusammen einen anellierten, gegebenenfalls substituierten Phenylen-1,2-Rest bilden, ein Verfahren zu ihrer Herstellung und ihre Verwendung zur Herstellung von Azalactonen.

EP 0 191 345 A2

BAYER AKTIENGESELLSCHAFT 5090 Leverkusen, Bayerwerk
Konzernverwaltung RP
Patentabteilung B/by-c

# Neue Cycloacetale, Verfahren zu ihrer Herstellung und ihre Verwendung zur Herstellung von Azalactonen

Cycloacetale sind bekannt; so sind z.B. bicyclische Acetale des Glyoxals in Chem. Ber. 1954, S. 1343 und Tetrahedron. Vol. 27 S. 5579 beschrieben. Sie werden durch Umsetzung von Glyoxal mit Glykolen, z.B. Ethylenglykol, erhalten.

Die Erfindung betrifft dagegen einen völlig neuartigen Cycloacetal-Typ, nämlich polycyclische Acetale (4H, 9H-Octahydro-1.4-dioxano/2.3-b:5.6-b'/bis/1.4/oxazine) der Formel

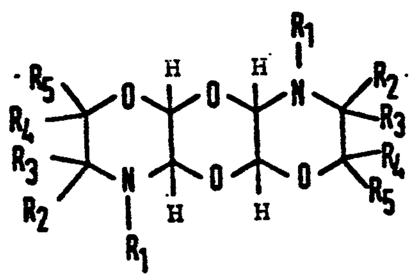

(I)

in der

$R_1$ für einen gegebenenfalls substituierten Alkyl-, Cycloalkyl-, Aralkyl-, Aryl- oder Hetaryl-Rest, vorzugsweise für einen gegebenenfalls substituierten

$C_1$-$C_{12}$-Alkyl-, Cyclopentyl-, Cyclohexyl-, Phenyl-$C_1$-$C_4$-alkyl- oder Phenyl-Rest, steht und

$R_2$, $R_3$, $R_4$ und $R_5$ unabhängig voneinander Wasserstoff, einen gegebenenfalls substituierten Alkyl-, Cycloalkyl-, Aralkyl-, Aryl- oder Hetaryl-Rest, vorzugsweise Wasserstoff, einen $C_1$-$C_{12}$-Alkyl-Rest oder einen gegebenenfalls substituierten Cyclopentyl-, Cyclohexyl-, Phenyl-$C_1$-$C_4$-alkyl- oder Phenyl-Rest bedeuten; oder $R_3$ und $R_4$ zusammen einen gegebenenfalls substituierten $C_2$-$C_6$-Alkylen-Rest, vorzugsweise einen $C_3$-$C_4$-Alkylen-Rest, bilden oder $R_2$, $R_3$, $R_4$ und $R_5$ zusammen einen anellierten, gegebenenfalls substituierten Phenylen-1,2-Rest bilden.

Für $R_1$, $R_2$, $R_3$, $R_4$ und $R_5$ seien genannt:

als gegebenenfalls substituierte Alkylreste: $C_1$-$C_{12}$-Alkylreste wie der Methyl-, Ethyl-, Propyl-, sec.-Butyl-, tert.-Butyl-, n-Pentyl-, n-Hexyl-, 2-Ethylhexyl-Rest und durch Halogenatome z.B. durch Fluor- und Chloratome substituierte Alkylreste, wie der Trichlormethyl-, Fluormethyl-, Difluormethyl- und Trifluormethyl-Rest; ferner durch Heterocyclen, z.B. durch den Furyl-, Imidazolyl-, oder Triazolyl-Rest substituierte Alkylreste, wie der Furfuryl-, 2-(Imidazolyl-(2))-ethyl- und 2-(Triazolyl-(2))-ethyl-Rest; ferner durch Alkoxy- oder Alkylmercapto-Gruppen substituierte Alkylreste,

als gegebenenfalls substituierte Cycloalkylreste: $C_5$-$C_7$-Cycloalkylreste wie der Cyclopentyl- und der

Cyclohexyl-Rest und die durch $C_1$-$C_4$-Alkylgruppen und/oder Halogenatome, z.B. Chlor oder Fluor, substituierten Cyclopentyl- oder Cyclohexyl-Reste wie der Methylcyclohexyl-, Dimethylcyclohexyl-, tert.-Butylcyclohexyl-Rest und halogenierte, vorzugsweise chlorierte und/oder fluorierte $C_5$-$C_7$-Cycloalkylreste wie der Chlorcyclohexyl-, Dichlorcyclohexyl- und der Trichlormethylcyclohexyl-Rest;

als gegebenenfalls substituierte Aralkylreste: Phenyl-$C_1$-$C_4$-alkyl-Reste wie der Benzyl-, α-Methylbenzyl-, der 2-, 3- oder 4-Methylbenzyl-, Chlorbenzyl-, Dichlorbenzyl-, Trifluormethylbenzyl- und der ß-Phenylethyl-Rest;

als gegebenenfalls substituierte Arylreste: vor allem einkernige aromatische Arylreste wie der Phenyl-, 4-Chlorphenyl-, 4-Cyanphenyl-, 4-Fluorphenyl-, der Tolyl- und der Xylyl-Rest;

als gegebenenfalls substituierte Hetarylreste: der Benzimidazol-, Benzotriazol-, Benzothiazol-, Phenothiazin-, Indol-, Carbazol-, Benzofuran- und der Chinolin-Rest.

Als zusammen von $R_3$ und $R_4$ zu bildende, gegebenenfalls substituierte $C_2$-$C_6$-Alkylenreste seien beispielsweise genannt: insbesondere $C_3$-$C_4$-Alkylenreste wie der Propylen-1,3- und der Butylen-1,4-Rest; als Substituenten dieser Alkylenreste kommen vor allem niedere Alkylgruppen wie die Methyl- und Ethylgruppe in Betracht.

Als Substituenten für den zusammen von $R_2$, $R_3$, $R_4$ und $R_5$ gebildeten, anellierten Phenylen-1,2-Rest kommen vor allem niedere Alkylgruppen wie die Methyl- oder tert.-Butylgruppe, Halogenatome wie Chlor oder Fluor, ferner die Cyan- und die Nitrogruppe in Betracht.

Als Vertreter der erfindungsgemäßen tricyclischen Acetale der Formel (I) seien beispielsweise genannt:

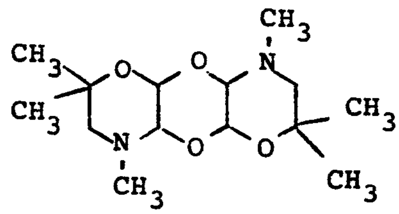

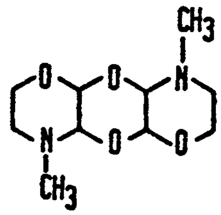

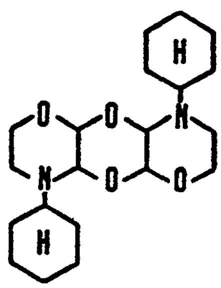

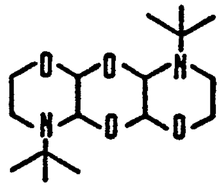

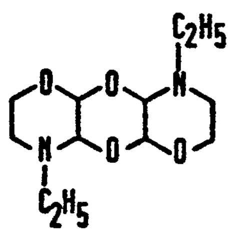

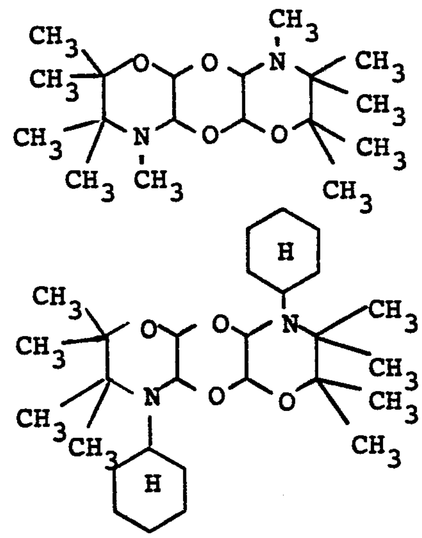

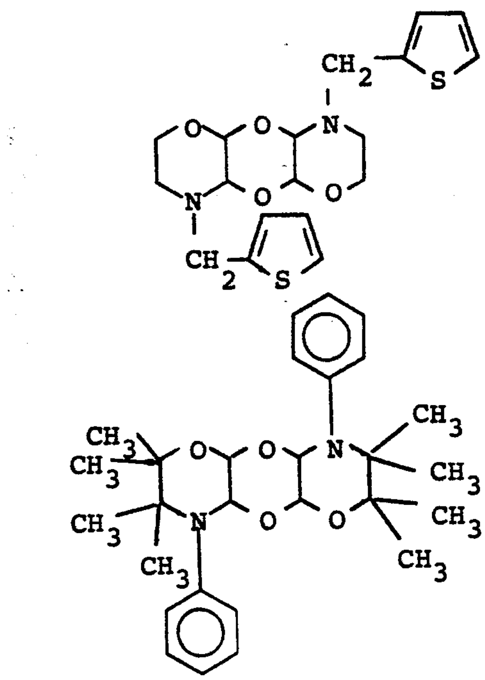

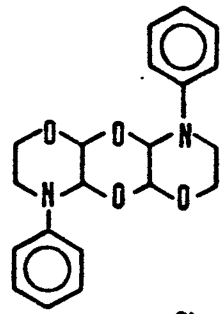

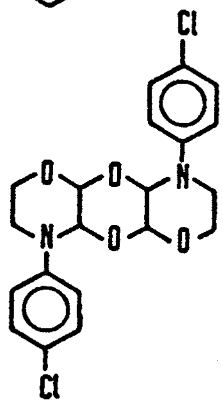

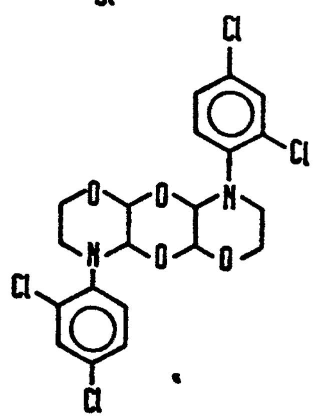

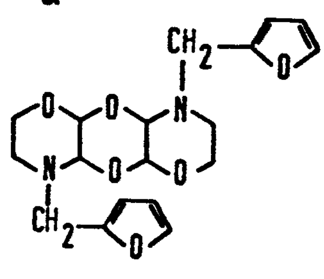

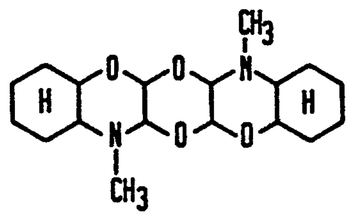

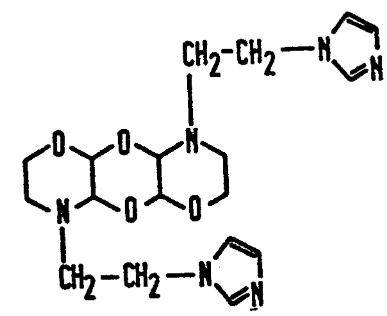

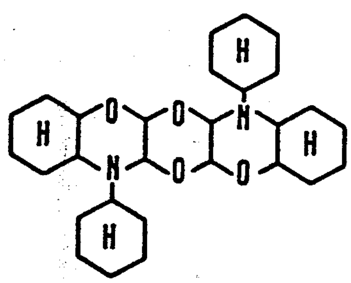

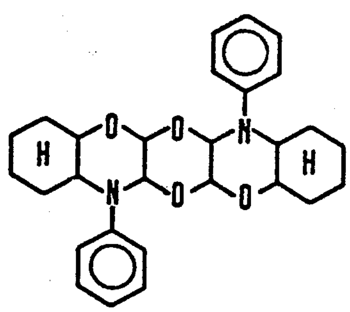

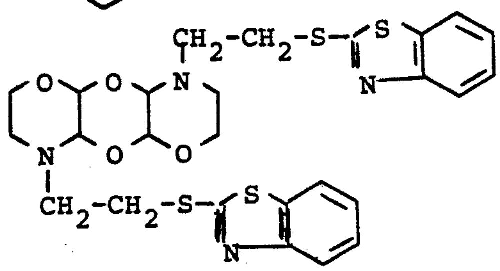

0191345



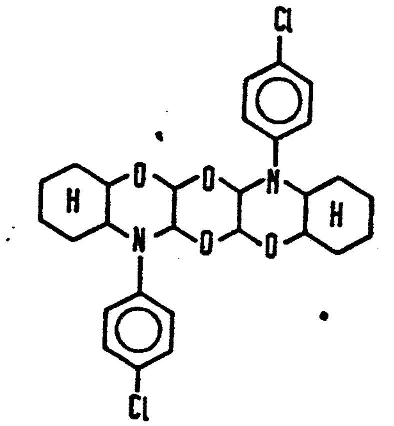

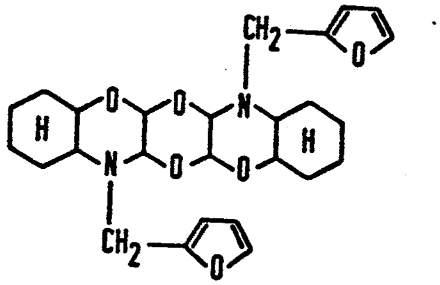

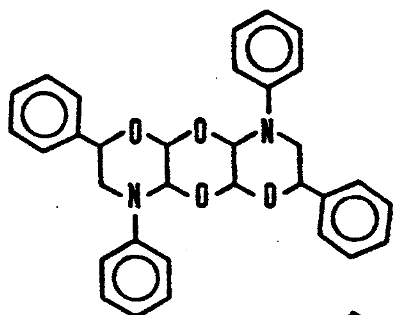

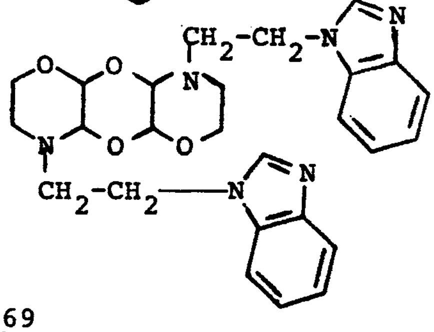

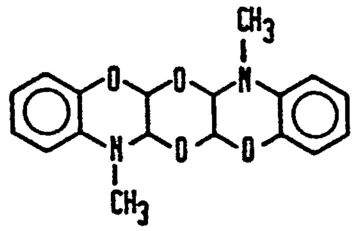

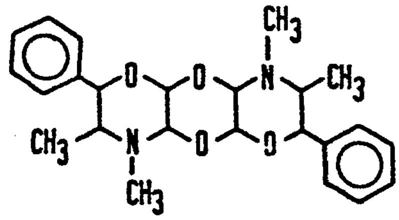

Die erfindungsgemäßen polycyclischen Acetale der Formel (I) werden durch Umsetzung von 2,3-Dihydroxydioxan mit 1,2-Aminoalkoholen erhalten. Die Erfindung betrifft daher auch ein Verfahren zur Herstellung von polycyclischen Acetalen der Formel (I), das dadurch gekennzeichnet ist, daß man 2,3-Dihydroxydioxan mit 1,2-Aminoalkoholen der Formel

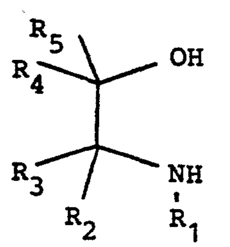

(II)

in der

$R_1$, $R_2$, $R_3$, $R_4$ und $R_5$ die unter Formel (I) angegebene Bedeutung haben,

vorzugsweise in einem inerten polaren Lösungsmittel umsetzt.

Die erfindungsgemäße Umsetzung des 2,3-Dihydroxydioxans mit den 1,2-Aminoalkoholen läßt sich durch die allgemeine Reaktionsgleichung beschreiben:

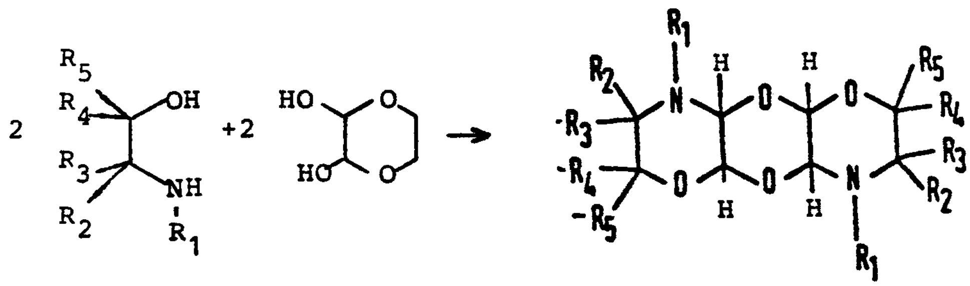

$+ 2\ HO-CH_2-CH_2-OH \quad + 2H_2O$

Als Vertreter der erfindungsgemäß verwendbaren 1,2-Aminoalkohole der Formel (II) seien beispielsweise genannt: 2-N-Methylamino-ethanol-(1), 2-N-Cyclohexylamino-ethanol-(1), 2-N-tert. Butylamino-ethanol-(1), 2-N-Ethylamino-ethanol-(1), 2-N-Phenylamino-ethanol-(1), 2-(N-4-Chlorphenyl)-amino-ethanol-(1), 2-(N-Furfuryl)-amino-ethanol-(1), 2-N-Methylamino-cyclohexanol-(1), 2-N-Cyclohexylamino-cyclohexanol-(1), 2-N-Phenylaminocyclohexanol-(1), 2-N-Chlorphenylamino-cyclohexanol-(1), 2-N-Furfurylamino-cyclohexanol-(1), 2-N-Methylaminocyclopentanol-(1), 2-N-Cyclohexylamino-cyclopentanol-(1), 2-N-Phenylamino-cyclopentanol-(1), 2-N-Chlorphenylaminocyclopentanol-(1), 2-N-Furfurylamino-cyclopentanol-(1), 2-N-Cyclopentylamino-cyclopentanol-(1), 2-N-Methylaminophenol, 2-N-Cyclohexylamino-phenol, 2-N-Phenylaminophenol.

Die Umsetzung des 2,3-Dihydroxydioxans mit den 1,2-Aminoalkoholen wird bei Temperaturen von 30 bis 150°C, vorzugsweise 60 bis 130°C, vorgenommen. 2,3-Dihydroxydioxan und 1,2-Aminoalkohole werden vorzugsweise in äquimolaren Mengen eingesetzt.

Als unter den Reaktionsbedingungen inerte polare Lösungsmittel werden vorzugsweise Wasser, Dimethylformamid, Dimethylacetamid, Dioxan oder Dimethylsulfoxid verwendet.

Die Umsetzung wird vorzugsweise so durchgeführt, daß man das 2,3-Dihydroxydioxan in dem vorgesehenen Lösungsmittel, z.B. Wasser oder Dimethylformamid, löst und diese Lösung mit dem 1,2-Aminoalkohol versetzt. Anschließend wird die Reaktionsmischung zur Vervollständigung der Reaktion gegebenenfalls noch eine Weile erwärmt. Beim Abkühlen scheiden sich die polycyclischen Cycloacetale der Formel (I) meistens in Form eines kristallinen Niederschlages ab. Sie werden in üblicher Weise, z.B. durch Filtrieren oder Zentrifugieren von der flüssigen Phase abgetrennt.

Die erfindungsgemäßen polycyclischen Acetale der Formel (I) eröffnen einen neuen, besonders einfachen Weg zur Herstellung von Azalactonen der Formel (III)

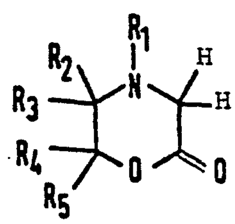

(III)

in der

$R_1$, $R_2$, $R_3$, $R_4$ und $R_5$ die unter Formel (I) angegebene Bedeutung haben.

Es wurde nämlich gefunden, daß sich die erfindungsgemäßen polycyclischen Acetale der Formel (I) beim Erhitzen über ihren Schmelzpunkt in hohen Ausbeuten in die Azalactone der Formel (III) spalten. Die Thermolyse der cyclischen Acetale der Formel (I) läßt sich durch die folgende Reaktionsgleichung veranschaulichen

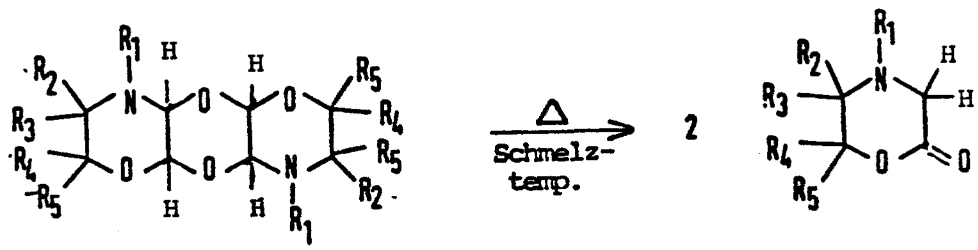


Die Thermolyse kann sowohl unter Normaldruck als auch unter vermindertem Druck vorgenommen werden.

Einige der Azalactone der Formel (III) sind bekannt; sie sind wichtige Ausgangsverbindungen für die Herstellung physiologisch wirksamer dihydroxylierter Diphenylalkylamine (siehe Arch. Pharm. 316/83 Seite 339 u.f.).

Diese Azalactone wurden bislang durch Umsetzung von Sarkosin mit Epoxiden erhalten. Dieses Herstellungsverfahren, obgleich es in recht guten Ausbeuten verläuft, hat jedoch den Nachteil, daß es nicht universell, d.h. zur Herstellung beliebig substituierter Azalactone,

anwendbar ist, weil die z.B. für die Herstellung durch Heteroatome, wie Stickstoff und/oder Schwefel, substituierter Azalactone erforderlichen Epoxide nicht zugänglich sind.

Im Gegensatz zur Synthese der Azalactone aus Sarkosin und Epoxiden, stellt die Thermolyse der erfindungsgemäßen polycyclischen Acetale der Formel (I) ein einfaches, vielseitiger anwendbares Verfahren zur Herstellung beliebig substituierter Azalactone aus einfach zugänglichen Vorstufen dar.

Die Erfindung betrifft daher auch die Verwendung der erfindungsgemäßen polycyclischen Lactone der Formel (I) als Zwischenprodukte für die Herstellung von Azalactonen der Formel (III). Als Vertreter der erfindungsgemäß herstellbaren Azalactone der Formel (III) seien beispielsweise genannt:

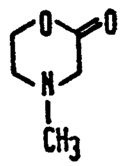
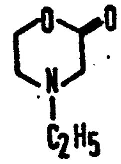
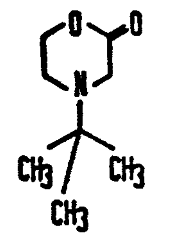

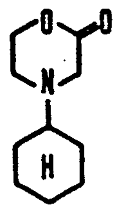
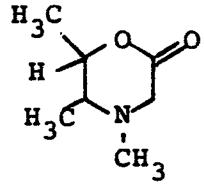
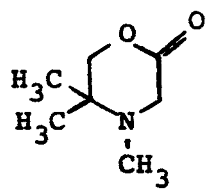

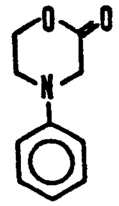
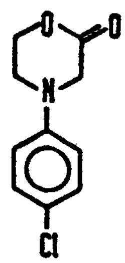
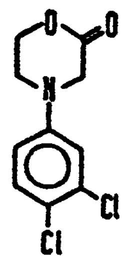
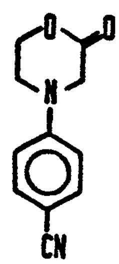

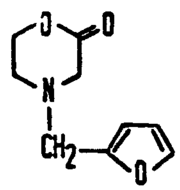
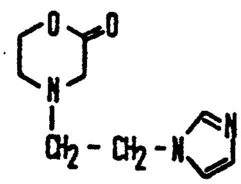
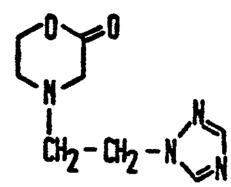

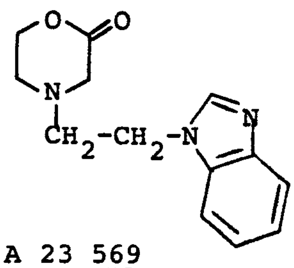
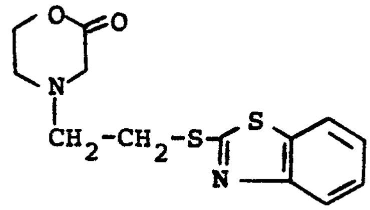

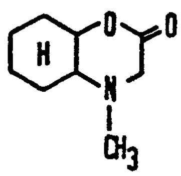
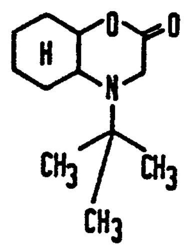
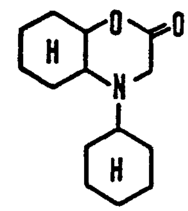

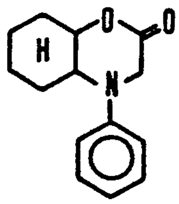
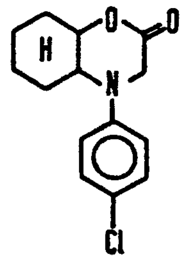
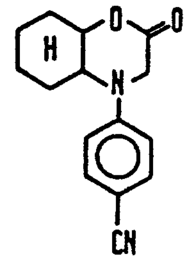

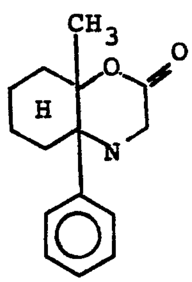
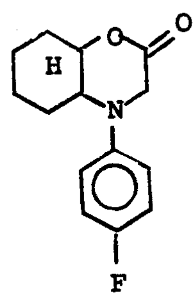
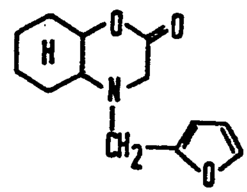

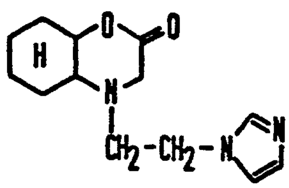
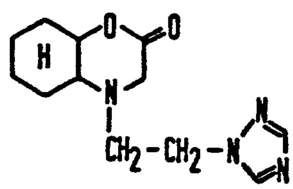
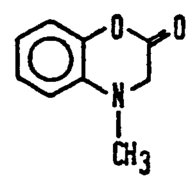

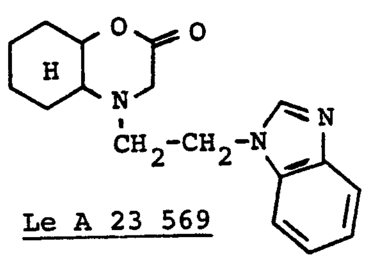
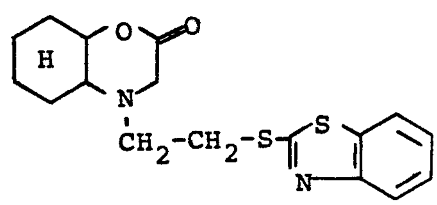

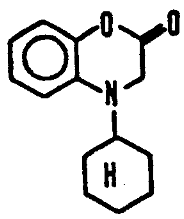

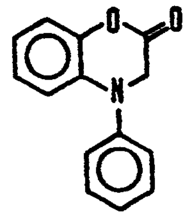

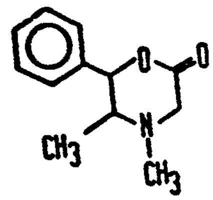

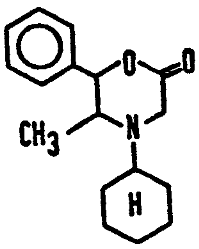

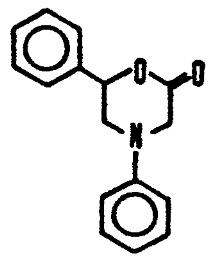

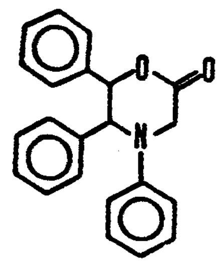

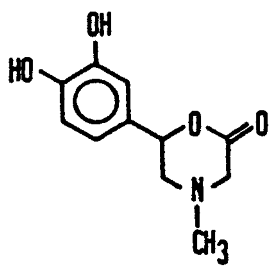

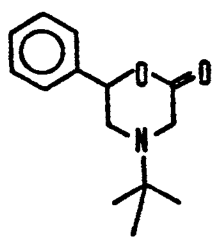

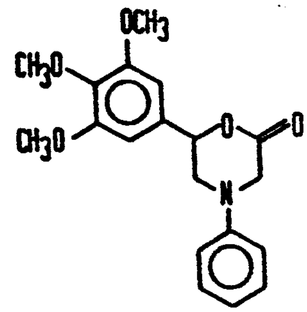

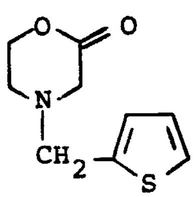

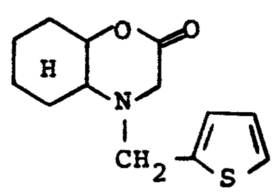

Die erfindungsgemäße Umsetzung der 2,3-Dihydroxydioxane mit den 1,2-Aminoalkoholen ist unabhängig von der Natur der Substituenten $R_1$, $R_2$, $R_3$, $R_4$ und $R_5$ des Aminoalkohols. Grundsätzlich ist jeder beliebig substituierte Aminoalkohol einsetzbar, in dem sich Amino- und Hydroxygruppe in 1,2-Stellung zueinander befinden. Daraus erklärt sich die große Anwendungsbreite des erfindungsgemäßen Verfahrens

a) zur Herstellung der polycyclischen Cycloacetale der Formel (I) und

b) zur Herstellung der Azalactone der Formel (III).

Mit Hilfe der erfindungsgemäßen Umsetzung des 2,3-Dihydroxydioxans mit den 1,2-Aminoalkoholen der Formel (II) zu den polycyclischen Acetalen der Formel (I) und die Thermolyse dieser cyclischen Acetale der Formel (I) zu Azalactonen der Formel (III) werden nicht nur bekannte Azalactone auf eine einfachere Weise zugänglich, sondern es wird die Herstellung einer Vielzahl neuer Azalactone möglich, und zwar durch eine einfache Reaktion, die auch in technischem Maßstab durchführbar ist.

Beispiel 1

a) Herstellung des 4.9-Dimethyl-4H,9H-octahydro-1.4-dioxano[2.3-b;5.6-b']bis[1.4]oxazins (1a)

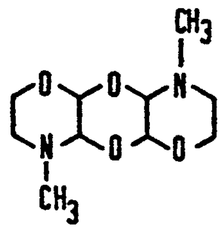

Die Lösung von 240 g (2 Mol) 2,3-Dihydroxydioxan in 300 ml Dimethylformamid wird bei Raumtemperatur tropfenweise mit 150 g 2-N-Methylamino-ethanol-(1) versetzt; dabei steigt die Temperatur der Reaktionsmischung auf 30°C. Anschließend wird die Reaktionsmischung 2 Stunden gerührt. Anschließend wird der kristalline Niederschlag abgesaugt und in Essigester aufgekocht. Es werden 225 g (= 98 % der Theorie) der Verbindung (1a) in Form farbloser Kristalle vom Schmelzpunkt 176°C (Zers.) erhalten.

Summenformel $C_{10}H_{18}N_2O_4$, Molekulargewicht: 230.
Analysendaten:
ber. C: 52,2 %, H: 7,8 %, N: 12,1 %;
gef. C: 52,3 %, H: 7,7 %, N: 12,1 %.

b) Thermolyse zum 4-N-Methylmorpholin-2-on

50 g der Verbindung (1a) werden in einem mit Rührer verschlossenen Kolben solange auf 175°C erhitzt,

bis sich das gesamte Produkt verflüssigt hat. Anschließend wird das flüssige Produkt abgekühlt und im Vakuum destilliert. Es werden 48 g (= 96 % der Theorie) 4-N-Methylmorpholin-2-on in Form einer farblosen Flüssigkeit (Kp: 56-58°C bei 0,09 mbar) erhalten.

Summenformel $C_5H_9NO_2$, Molekulargewicht: 115.
Analysendaten:
ber. C: 52,2 %, H: 7,8 %, N: 12,1 %;
gef. C: 52,2 %, H: 7,8 %, N: 12,1 %.

Beispiel 2

a) Herstellung des 4.9-Dicyclohexyl-4H,9H-octahydro-1.4-dioxano/2.3-b:5.6-b'/bis/1.4/oxazins (2a)

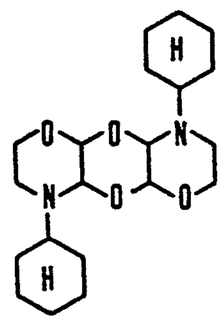

Die Lösung von 48 g (0,4 Mol) 2,3-Dihydroxydioxan in 60 ml Dimethylformamid wird bei Raumtemperatur unter Rühren mit der Lösung von 57,2 g (0,4 Mol) 2-N-Cyclohexylamino-ethanol-(1) in 20 ml Dimethylformamid versetzt. Es entsteht eine klare Lösung, aus der sich nach etwa einstündigem Rühren ein kristalliner Niederschlag abscheidet. Der Niederschlag wird

abgesaugt und mit 100 ml Essigester aufgekocht. Es werden 66 g (= 90 % der Theorie) der Verbindung (2a) in Form farbloser Kristalle vom Schmelzpunkt 174-177°C (Zers.) erhalten.

Summenformel $C_{20}H_{34}N_2O_4$, Molekulargewicht: 366.
Analysendaten:
ber. C: 65,5 %, H: 9,3 %, N: 7,6 %;
gef. C: 64,8 %, H: 9,0 %, N: 7,5 %.

b) Thermolyse zum 4-N-Cyclohexylmorpholin-2-on

36,6 g der Verbindung (2a) werden in einem mit Rührer verschlossenen Kolben solange auf 190°C erwärmt, bis sich die gesamte Masse verflüssigt hat. Die Flüssigkeit wird abgekühlt und im Vakuum destilliert. Es werden 33 g (= 90 % der Theorie) 4-N-Cyclohexylmorpholin-2-on in Form einer farblosen Flüssigkeit (Kp.: 116°C bei 0,1 mbar) erhalten.

Summenformel $C_{10}H_{17}NO_2$, Molekulargewicht: 183.
Analysendaten:
ber. C: 65,5 %, H: 9,3 %, N: 7,6 %;
gef. C: 65,0 %, H: 9,3 %, N: 7,8 %.

Beispiel 3

a) Herstellung des 4.9-Diphenyl-4H,9H-octahydro-1.4-dioxano[2.3-b:5.6-b']bis[1.4]oxazins (3a)

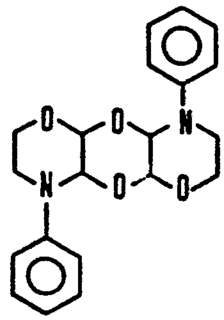

Die Lösung von 48 g (0,4 Mol) 2,3-Dihydroxydioxan in 50 ml Dimethylformamid wird bei Raumtemperatur tropfenweise unter Rühren mit 54,8 g (0,4 Mol) N-2-Hydroxyethyl-anilin versetzt. Die Lösung wird 1 Stunde auf 80°C erwärmt. Nach dem Abkühlen wird der Niederschlag abgesaugt und mit Dioxan aufgekocht. Es werden 60 g (= 84 % der Theorie) der Verbindung (3a) in Form farbloser Kristalle vom Schmelzpunkt 255-258°C (Zers.) erhalten.

Summenformel $C_{20}H_{22}N_2O_4$, Molekulargewicht: 354.
Analysendaten:
ber. C: 67,7 %, H: 6,2 %, N: 7,9 %;
gef. C: 67,9 %, H: 6,3 %, N: 8,1 %.

b) Thermolyse zum 4-N-Phenylmorpholin-2-on (3b)

35,4 g (0,1 Mol) der Verbindung (3a) werden in einem mit Rührer verschlossenen Kolben solange auf 240°C erhitzt, bis eine klare Flüssigkeit entstanden ist. Die Flüssigkeit wird abgekühlt und anschließend im

Vakuum destilliert. Es werden 30 g (= 84 % der Theorie) 4-N-Methylmorpholin-2-on in Form einer klaren Flüssigkeit (Kp.: 146-156°C bei 0,2 mbar) erhalten, die beim Abkühlen schnell in Form farbloser Blättchen kristallisiert. Fp.: 71-73°C.

Summenformel $C_{10}H_{11}NO_2$.
Analysendaten:
ber. C: 67,7 %, H: 6,2 %, N: 7,9 %;
gef. C: 67,7 %, H: 6,1 %, N: 7,9 %.

Beispiel 4

a) Herstellung des 4.9-Dimethyl-2.3.7.8-bis-tetramethylen-4H,9H-octahydro-1.4-dioxano/2.3-b:5.6-b'/-bis/1.4/oxazins (4a)

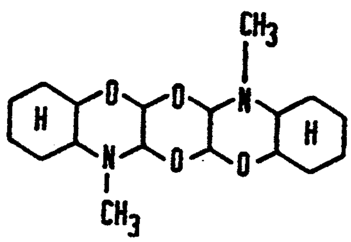

Die Lösung von 48 g (0,4 Mol) 2,3-Dihydroxydioxan in 40 ml Dimethylformamid wird bei Raumtemperatur unter Rühren mit 51,6 g (0,4 Mol) 2-N-Methylamino-cyclohexanol-(1) versetzt. Die Lösung wird 5 Stunden auf 80°C erwärmt. Beim Abkühlen kristallisiert aus der Lösung ein farbloser Niederschlag aus. Es werden 59 g (= 86 % der Theorie) der Verbindung (4a) in Form farbloser Kristalle vom Schmelzpunkt 242-245°C (Zers.) erhalten.

Summenformel $C_{18}H_{30}N_2O_4$, Molekulargewicht: 338.
Analysendaten:
ber. C: 63,7 %, H: 8,8 %, N: 8,3 %;
gef. C: 63,6 %, H: 8,3 %, N: 8,5 %.

b) Thermolyse zum 4-N-Methyl-5.6-tetramethylen-morpholin-2-on (4b)

33,8 g ( 0,1 Mol) der Verbindung (4a) werden solange auf 250°C erhitzt, bis eine klare Flüssigkeit entstanden ist. Die Flüssigkeit wird abgekühlt und im Vakuum destilliert. Es werden 28 g (= 83 % der Theorie) an 4-N-Methyl-5.6-tetramethylenmorpholin-2-on in Form eines leicht viskosen Öls (Kp.: 99-101°C bei 0,09 mbar) erhalten.

Summenformel $C_9H_{15}NO_2$, Molekulargewicht: 169.
Analysendaten:
ber. C: 63,7 %, H: 8,8 %, N: 8,3 %;
gef. C: 63,6 %, H: 8,5 %, N: 8,6 %.

Beispiel 5

a) Herstellung des 4.9-Bisfurfuryl-2.3.7.8-bis-tetramethylen-4H,9H-octahydro-1.4-dioxano/2.3-b:5.6-b'/-bis/1.4/oxazins (5a)

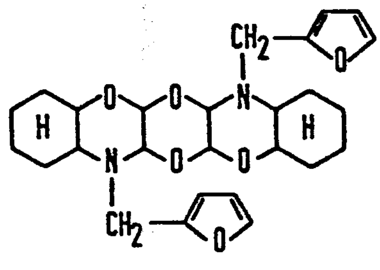

Die Lösung von 48 g (0,4 Mol) 2,3-Dihydroxydioxan in 40 ml Dimethylformamid wird bei Raumtemperatur unter Rühren mit 78 g (0,4 Mol) 2-N-Furfurylamino-cyclohexanol-(1) versetzt. Die Lösung wird 2 Stunden auf 80°C erwärmt. Nach dem Abkühlen wird der auskristallisierte Niederschlag abgesaugt und in Essigester aufgekocht. Es werden 80,6 g (= 85 % der Theorie) der Verbindung (5a) in Form farbloser Kristalle vom Schmelzpunkt 221-226°C (Zers.) erhalten.

Summenformel $C_{26}H_{34}N_2O_6$, Molekulargewicht: 470.
Analysendaten:
ber. C: 66,3 %, H: 7,2 %, N: 5,9 %;
gef. C: 66,4 %, H: 7,1 %, N: 5,9 %.

b) Thermolyse zum 4-N-Furfuryl-5.6-tetramethylen-morpholin-2-on (5b)

47 g (0,1 Mol) der Verbindung (5a) werden solange auf 230°C erwärmt, bis eine klare Flüssigkeit entstanden ist. Die Flüssigkeit wird abgekühlt und im Vakuum destilliert. Es werden 39 g (= 83 % der Theorie) 4-N-Furfuryl-5.6-tetramethylen-morpholin-2-on in Form einer klaren Flüssigkeit (Kp.: 172-177°C bei 0,14 mbar) erhalten.

Summenformel $C_{13}H_{17}NO_3$, Molekulargewicht: 235.
Analysendaten:
ber. C: 66,3 %, H: 7,2 %, N: 5,9 %;
gef. C: 66,3 %, H: 7,2 %, N: 5,8 %.

Beispiel 6

a) Herstellung des 2.7-Diphenyl-3.4.8.9-tetramethyl-4H,9H-octahydro-1.4-dioxano-/2.3-b:5.6-b'/bis/1.4/-oxazins (6a)

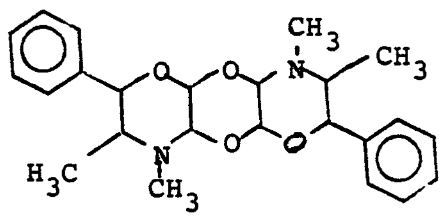

Die Lösung von 48 g (0,4 Mol) 2,3-Dihydroxydioxan in 50 ml Dimethylformamid wird bei Raumtemperatur unter Rühren mit 64 g (0,4 Mol) l(-)-Ephedrin versetzt. Die Lösung wird 2 Stunden auf 70°C erhitzt. Anschließend wird das Reaktionsgemisch im Hochvakuum von allen flüchtigen Verbindungen befreit. Der Destillationsrückstand kristallisiert langsam. Das kristalline Produkt wird auf Ton getrocknet. Es werden 60 g (= 73 % der Theorie) der Verbindung 6a in Form einer kristallinen Masse vom Schmelzpunkt 175°C (Zers. erhalten.

Summenformel: $C_{24}H_{30}N_2O_4$
Molekulargewicht: 410
Analysendaten:
ber.: C: 70,2 %, H: 7,3 %, N: 6,8 %;
gef.: C: 69,0 %, H: 7,4 %, N: 6,5 %;

b) Thermolyse zum 4.5-Dimethyl-6-phenyl-morpholin-2-on (6b)

4,1 g (0,1 Mol) der Verbindung (6a) werden solange auf 180°C erhitzt, bis eine klare Flüssigkeit entstanden ist. Die Flüssigkeit wird abgekühlt und im Vakuum destilliert. Es werden 3,5 g (= 85 % der Theorie) 4.5-Dimethyl-6-phenyl-morpholin-2-on in Form einer farblosen Flüssigkeit vom Kp.: 128 bis 134°C bei 0,02 mbar) erhalten.

Summenformel $C_{12}H_{15}NO_2$,
Analysendaten:
ber. C: 70,2 %, H: 7,3 %, N: 6,8 %;
gef. C: 69,5 %, H: 7,4 %, N: 7,2 %.

Beispiel 7

Herstellung des 4.9-Bis-tert.-butyl-4H,9H-ocatahydro-1.4-dioxano/2.3-b:5.6-b'/bis/1.4/oxazins (7a)

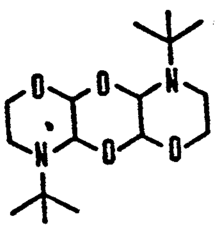

Die Lösung von 24 g (0,2 Mol) 2,3-Dihydroxydioxan in 30 ml Dimethylformamid wird bei Raumtemperatur unter Rühren tropfenweise mit der Lösung von 23,4 g (0,2 Mol) 2-N-Butylamino-ethanol-(1) in 10 ml Dimethylformamid versetzt. Die Lösung wird 2 Stunden auf 50°C erwärmt und über Nacht aufbewahrt. Der Niederschlag wird abgesaugt und aus Dimethylformamid umkristallisiert. Es werden 25,4 g (= 80 % der Theorie) der Verbindung (7a) vom Schmelzpunkt 170-173°C (Zers.) erhalten.

Summenformel $C_{16}H_{30}N_2O_4$, Molekulargewicht: 311.
Analysendaten:
ber. C: 61,1 %, H: 9,6 %, N: 8,9 %;
gef. C: 61,2 %, H: 9,1 %, N: 8,5 %.

Beispiel 8

Herstellung des 4.9-Bis-(4-chlorphenyl)-4H,9H-octahydro-1.4-dioxano[2.3-b:5.6-b']bis[1.4]oxazins (8)

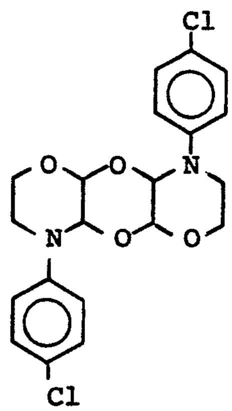

Die Lösung von 24 g (0,2 Mol) 2,3-Dihydroxydioxan in 50 ml Dimethylformamid wird bei Raumtemperatur unter Rühren mit 34,2 g (0,2 Mol) N-(4-Chlorphenyl)-ethanolamin versetzt. Die Lösung wird eine Stunde auf 70° erwärmt und anschließend im Vakuum von allen flüchtigen Verbindungen befreit. Der Rückstand wird aus Dimethylformamid umkristallisiert. Es werden 28 g (= 65 % der Theorie) der Verbindung (8) vom Schmelzpunkt 279-282°C (Zers.) erhalten.

Summenformel $C_{20}H_{20}Cl_2N_2O_4$, Molekulargewicht 423
Analysendaten:
ber.: C: 56,8 %, H: 4,7 %, N: 6,6 %, Cl: 16,8 %
gef.: C: 57,0 %, H: 4,8 %, N: 6,5 %, Cl: 16,8 %.

Beispiel 9

Herstellung des 4.9-Bis-(4-Chlorphenyl)-2.3.7.8-bis-tetramethylen-4H,9H-octahydro-1.4-dioxano/2.3-b:5.6-b'/-bis/1.4/oxazins (9)

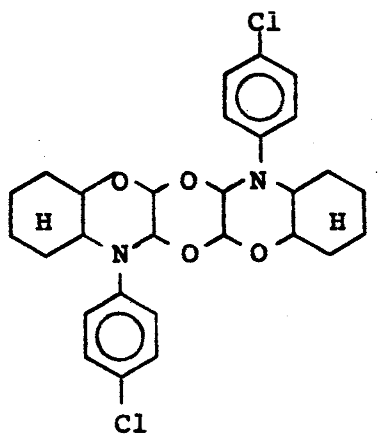

Die Lösung von 24 g (0,2 Mol) 2,3-Dihydroxydioxan in 50 ml Dimethylformamid wird unter Rühren mit 44,4 g (0,2 Mol) 2-N-(4-Chlorphenyl)-aminohexanol-(1) versetzt. Die Lösung wird 3 Stunden auf 100°C erwärmt. Nach dem Abkühlen wird der Niederschlag abgesaugt. Es werden 25 g

(= 47 % der Theorie) der Verbindung (9) in Form farbloser Kristalle vom Schmelzpunkt 291-293°C erhalten.

Summenformel $C_{28}H_{32}Cl_2N_2O_4$, Molekulargewicht: 531
Analysendaten:
ber.: C: 63,3 %, H: 6,0 %, N: 5,2 %, Cl: 13,4 %
gef.: C: 62,7 %, H: 6,1 $, N: 5,2 %, Cl: 13,4 %.

Patentansprüche:

1. Polycyclische Acetale der Formel

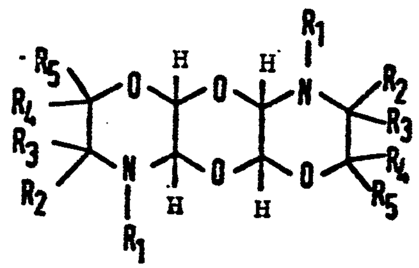

in der

$R_1$ für einen gegebenenfalls substituierten Alkyl-, Cycloalkyl-, Aralkyl-, Aryl- oder Hetaryl-Rest steht und

$R_2$, $R_3$, $R_4$ und $R_5$ unabhängig voneinander Wasserstoff, einen gegebenenfalls substituierten Alkyl-, Cycloalkyl-, Aralkyl- oder Aryl-Rest bedeuten, oder $R_3$ und $R_4$ zusammen einen gegebenenfalls substituierten $C_2$-$C_6$-Alkylen-Rest bilden oder $R_2$, $R_3$, $R_4$ und $R_5$ zusammen einen anellierten, gegebenenfalls substituierten Phenylen-1,2-Rest bilden.

2. Polycyclische Acetale gemäß Anspruch 1, dadurch gekennzeichnet, daß

$R_1$ für einen gegebenenfalls substituierten $C_1$-$C_{12}$-Alkyl-, Cyclopentyl-, Cyclohexyl-, Phenyl-$C_1$-$C_4$-alkyl- oder Phenyl-Rest steht und

$R_2, R_3, R_4$ und $R_5$ unabhängig voneinander Wasserstoff, einen $C_1$-$C_{12}$-Alkyl-Rest oder einen gegebenenfalls substituierten Cyclopentyl-, Cyclohexyl-, Phenyl-$C_1$-$C_4$-alkyl- oder Phenyl-Rest bedeuten, oder $R_3$ und $R_4$ zusammen einen gegebenenfalls substituierten $C_3$-$C_4$-Alkylen-Rest bilden oder $R_2, R_3, R_4$ und $R_5$ zusammen einen annellierten gegebenenfalls substituierten Phenylen-1,2-Rest bilden.

3. Verfahren zur Herstellung von polycyclischen Acetalen der Formel

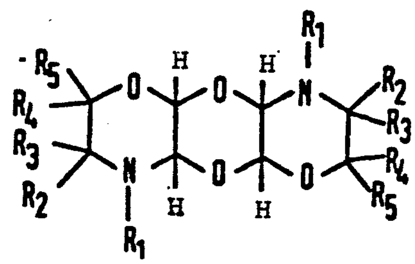

in der

$R_1$ für einen gegebenenfalls substituierten Alkyl-, Cycloalkyl-, Aralkyl-, Aryl- oder Hetaryl-Rest steht und

$R_2$, $R_3$, $R_4$ und $R_5$ unabhängig voneinander Wasserstoff, einen gegebenenfalls substituierten Alkyl-, Cycloalkyl-, Aralkyl- oder Aryl-Rest bedeuten, oder $R_3$ und $R_4$ zusammen einen gegebenenfalls

substituierten $C_2$-$C_6$-Alkylen-Rest bilden oder $R_2$, $R_3$, $R_4$ und $R_5$ zusammen einen anellierten, gegebenenfalls substituierten Phenylen-1,2-Rest bilden,

dadurch gekennzeichnet, daß man 2,3-Dihydroxydioxan mit 1,2-Aminoalkoholen der Formel

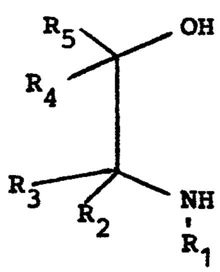

in der

$R_1$, $R_2$, $R_3$, $R_4$ und $R_5$ die angegebene Bedeutung haben,

vorzugsweise in einem inerten polaren Lösungsmittel umsetzt.

4. Verwendung der polycyclischen Acetale der Formel

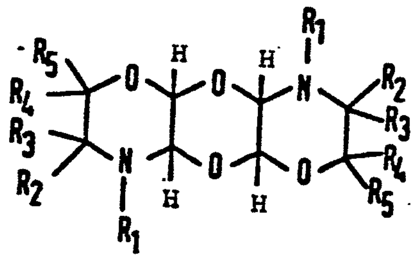

in der

$R_1$ für einen gegebenenfalls substituierten Alkyl-, Cycloalkyl-, Aralkyl-, Aryl- oder Hetaryl-Rest steht und

$R_2$, $R_3$, $R_4$ und $R_5$ unabhängig voneinander Wasserstoff, einen gegebenenfalls substituierten Alkyl-, Cycloalkyl-, Aralkyl- oder Aryl-Rest bedeuten, oder $R_3$ und $R_4$ zusammen einen gegebenenfalls substituierten $C_2$-$C_6$-Alkylen-Rest bilden oder $R_2$, $R_3$, $R_4$ und $R_5$ zusammen einen anellierten, gegebenenfalls substituierten Phenylen-1,2-Rest bilden,

zur Herstellung von Azalactonen der Formel

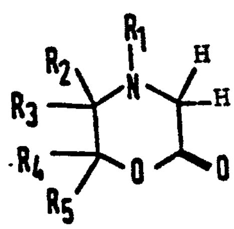

in der

$R_1$, $R_2$, $R_3$, $R_4$ und $R_5$ die vorstehend angegebene Bedeutung haben.